(19) Europäisches Patentamt
European Patent Office
Office européen des brevets

(11) Veröffentlichungsnummer: **0 022 910**
**A1**

(12) # EUROPÄISCHE PATENTANMELDUNG

(21) Anmeldenummer: **80102819.2**

(22) Anmeldetag: **21.05.80**

(51) Int. Cl.$^3$: **C 12 P 17/12**
C 07 D 211/88, A 01 N 43/40
//(C12P17/12, C12R1/545)

(30) Priorität: **26.05.79 DE 2921401**
**07.09.79 DE 2936238**

(43) Veröffentlichungstag der Anmeldung:
**28.01.81 Patentblatt 81/4**

(84) Benannte Vertragsstaaten:
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Berg, Dieter, Dr.**
**Claudiusweg 9**
**D-5600 Wuppertal 1(DE)**

(72) Erfinder: **Schedel, Michael, Dr.**
**Sillerstrasse 49**
**D-5600 Wuppertal 1(DE)**

(72) Erfinder: **Schmidt, Robert Rudolf, Dr.**
**Hahnenweg 5**
**D-5000 Köln 80(DE)**

(72) Erfinder: **Weyland, Horst, Dr.**
**Fichtestrasse 12**
**D-2850 Bremerhaven(DE)**

(54) **Mikrobiologische Verfahren zur Herstellung von 1R;3S;5S;alpha R;3-(2-(3,5-Dimethyl-2-oxocyclohexyl)-2-hydroxyethyl)-2,6-piperidindion, dessen Verwendung und für das Herstellungsverfahren verwendbare Mikroorganismen.**

(57) Gegenstand der Erfindung ist ein Verfahren zur Herstellung von 1R;3S;5S;αR;3-[2-(3,5-Dimethyl-2 oxocyclohexyl)-2-hydroxyethyl]-2,6-piperidindion der Formel

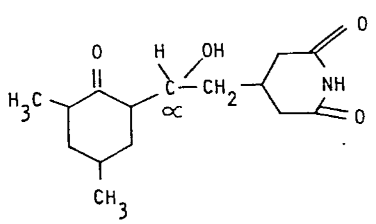

das dadurch gekennzeichnet ist, daß man Mikroorganismen der Ordnung Actinomycetales, insbesondere Streptomyces griseus Stamm 587 (DSM 1471) in einem Nährmedium, welches assimilierbare Kohlenstoff- und Stickstoffquellen sowie Mineralsalze enthält, unter aeroben Bedingungen, kultiviert und die Verbindung isoliert, sowie die Verwendung der so hergestellten Verbindungen als Pflanzenschutzmittel, insbesondere als Herbizid, Pflanzenwachstumsregulator und insbesondere als Mittel zur Erleichterung der Fruchtablösung.

FIG 1

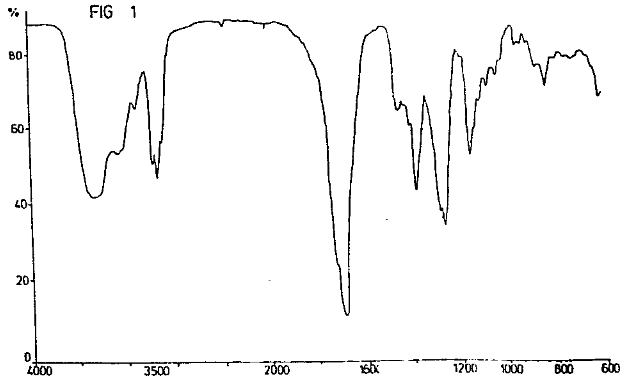


EP 0 022 910 A1

BAYER AKTIENGESELLSCHAFT 5090 Leverkusen, Bayerwerk
Zentralbereich
Patente, Marken und Lizenzen Ad/ABc

BEZEICHNUNG GEÄNDERT
siehe Titelseite

Mikrobiologische Verfahren zur Herstellung von 1R; 3S; 5S; α R; 3-[2-(3,5-Trimethyl-2-oxocyclohexyl)-2-hydroxyethyl]-2,6-piperidindion, und dessen Verwendung

Die vorliegende Erfindung betrifft mikrobiologische Verfahren zur Herstellung von 1R; 3S; 5S; α R; 3-[2-(3,5-Trimethyl-2-oxocyclohexyl)-2-hydroxyethyl]-2,6-piperidindion sowie dessen Verwendung als Pflanzenschutzmittel, insbesondere als Herbizid und Pflanzenwachstumsregulator.

Die eingangs genannte Verbindung ist bekannt (s. Chemical Abstracts Vol. 55, 21476f) und läßt sich durch das in Figur 1 wiedergegebene IR-KBr-Absorptionsspektrum (Abszisse: Wellenzahl in $cm^{-1}$, Ordinate: Extinktion), das charakteristische Banden bei den Wellenzahlen 825 $cm^{-1}$ aufweist, charakterisieren.

Des weiteren kann die Verbindung durch die folgenden Eigenschaften und Parameter beschrieben werden:

(1) Elementaranalyse
C: 63,2 % H: 8,0 % N: 4,7 % O⁺: 24,1 %
⁺aus der Differenz berechnet

(2) Zersetzungspunkt
Die Substanz bildet ein weißes amorphes Pulver mit einem Zersetzungspunkt von 120 - 125 °C.

(3) ORD-Spektrum (Fig. 5)
Das ORD-Spektrum der Substanz zeigt ein Maximum bei 297 nm (+ 380°C, c = 3 mg in 10 ml Chloroform)

Ordinate: Drehzahl in [°]
Abzisse: Wellenlänge in nm

| | |
|---|---|
| Kurve 1 | 4,2 mg/ml Chloroform |
| Kurve 2 | 0,42 mg/ml Chloroform |
| Kurve 3 | 0,84 mg/ml Chloroform |

(4) Ultraviolettabsorptionsspektrum
Im Bereich von 350 nm bis 200 nm zeigt die Verbindung keine Absorption.

(5) $^1$H-100 MHz-Kernresonanzspektrum
Das charakteristische $^1$H-NMR-Spektrum ist in Figur 2 wiedergegeben (Lösungsmittel $CDCl_3$).

(6) Massenspektrum (gemäß Figur 3)
Im Massenspektrum wird ein Molekülion nicht beobachtet. Es treten charakteristische Fragmentionen bei 263, 205, 204, 153, 152, 126, 112, 84, 69, 55 und 41 auf.

(7) Die Verbindung ist löslich in Methanol, Ethanol, Ethylacetat, Butylacetat, Benzol, Aceton und Chloroform, schwerlöslich in Wasser.

(8) Die Verbindung läßt sich sauer hydrolysieren. So können beispielsweise nach 14 Stunden langer Behandlung mit 6 N Salzsäure bei 110°C im Hydrolysat Ammoniumionen nachgewiesen werden.

Der Ersetzung dieser Seite 2 wird gemäss Regel 88, stattgegeben.
Den Haag, 7. August 1980
Eingangsstelle
P. J. MASSAAR

(9) Die Verbindung ist ein amorpher farbloser Stoff. Es ist leicht möglich, die Verbindung bei der Dünnschichtchromatographie auf einer Kieselgelplatte anzufärben. Einige Reaktionen, die zur Indentifikation dienen, sind in Tabelle 1 aufgeführt.

(10) Die $R_f$-Werte der Verbindung auf neutralen Kieselgelplatten gibt Tabelle 2 wieder.

(11) Die antibiotische Wirkung der erfindungsgemäßen Verbindung ist in Tabelle 3 wiedergegeben.

Tabelle 1: Versuche zur chemischen Anfärbung der Verbindung

| Nr. | Reagenz / Reaktion nach+) | Anfärbung |
|---|---|---|
| 1 | Ninhydrin | - |
| 2 | Morgan/Elson | - |
| 3 | 4-Dimethylaminobenzaldehyd | - |
| 4 | $KMnO_4$, alk. | + |
| 5 | Thymol/$H_2SO_4$ | + |
| 6 | Anisaldehyd/$H_2SO_4$ | + |
| 7 | Perjodat | - |
| 8 | 2,7-Dichlorfluorescein | - |
| 9 | Jod | + |

Tabelle 1 (Fortsetzung)

| Nr. | Reagenz / Reaktion nach +) | Anfärbung |
|---|---|---|
| 10 | Bromkresolgrün | - |
| 11 | Rhodamin B | - |
| 12 | Moybdatophosphorsäure | + |
| 13 | Anilinphthalat | - |
| 14 | Tollen's Reagenz | - |
| 15 | Diphenylamin | + |
| 16 | 2,4-Dinitrophenylhydrazin | - |
| 17 | Eisen(III)-chlorid | - |

+) Die Reagenzien wurden nach den üblichen Vorschriften (Vgl. E. Stahl, Dünnschichtchromatographie, 2. Auflage, Springer-Verlag, Berlin-Heidelberg-New York, 1967) angesetzt.

Tabelle 2: Dünnschichtchromatographische Charakterisierung der Verbindung auf Kieselgel 60, F 254, Schichtdicke 0,25 mm (Merck)

| Fließmittelsystem (Volumenteile) | $R_f$-Werte |
|---|---|
| n-Butanol/Eisessig/$H_2O$ = 50/25/25 | 0,60 |
| Chloroform/Methanol/Eisessig = 90/8/2 | 0,63 |
| Chloroform/Methanol/$H_2O$ = 80/20/2,5 | 0,66 |
| Aceton | 0,55 |
| Chloroform/Methanol = 80/20 | 0,65 |

Tabelle 2 (Fortsetzung)

| Fließmittelsystem (Volumenteile) | $R_f$-Werte |
|---|---|
| Chloroform/Methanol = 90/10 | 0,37 |
| Chloroform | 0,05 |
| Diethylether | 0,06 |
| Chloroform/Methanol/Ammoniak = 20/3/0,5 | 0,62 |
| n-Butylacetat/n-Butanol/Eisessig/Phosphatpuffer pH 7 = 50/10/25/15 | 0,62 |
| i-Propanol/2 N Ammoniak/$H_2O$ = 7/1/2 | 0,71 |
| Methanol | 0,64 |

Tabelle 3: Antibiotische Wirkung der Verbindung

| Organismus | minimale Hemmkonzentration /ug/ml |
|---|---|
| Hefe HT - 21 | ≦0,5 |
| Hefe HT - 10 | ≦0,5 |
| Sacharomyces cerevisiae | ≦1,0 |
| Pyricularia oryzae | ≦0,5 |
| Pellicularia sasakii | ≦1,0 |
| Sclerotinia sclerotiorum | ≦1,0 |
| Botrytis cinerea | ≦8,0 |
| Xanthomonas oryzae | >1000 |
| E. coli | >1000 |

1R;3S;5S; αR; 3-/2-(3,5- Dimethyl-2-oxocyclohexyl)-2-hydroxyethyl/-2,6-piperidindion hat die Formel

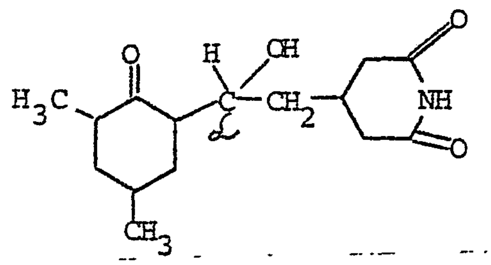

Diese Verbindung erhält man überraschenderweise, wenn man Mikroorganismen der Ordnung Actinomycetales in einem Nährmedium, welches assimilierbare Kohlenstoff- und Stickstoffquellen sowie Mineralsalze enthält unter aeroben Bedingungen kultiviert und die Verbinding isoliert.

Gegenstand der Erfindung ist daher ein Verfahren zur Herstellung von 1R;3S;5S;αR; 3-/2-(3,5- Dimethyl-2-oxocyclohexyl)-2-hydroxyethyl/-2,6-piperidindion, das dadurch gekennzeichnet ist, daß man Mikroorganismen der Ordnung Actinomycetales in einem Nährmedium, welches assimilierbare Kohlenstoff- und Stickstoffquellen sowie Mineralsalze enthält, unter aeroben Bedingungen kultiviert und die Verbindung isoliert, sowie die Verwendung der so hergestellten Verbindung als Pflanzenschutzmittel, insbesondere als Herbizid und Pflanzenwachstumsregulator.

Zur Gewinndung der Verbindung eignen sich innerhalb der Ordnung Actinomycetales besonders Vertreter der Familie Streptomycetaceae und hier hauptsächlich solche aus der Gattung Streptomyces. Das erfindungsgemäße Verfahren kann besonders gut mit Mikroorganismen der Art Streptomyces griseus durchgeführt werden. Als Beispiel sei Streptomyces griseus Stamm 587 genannt. Dieser Stamm wurde unter der Nummer DSM 1471 am 23. Februar 1979 bei der Deutschen Sammlung von Mikroorganismen Göttingen hinterlegt. Bakterienstämme, die zur Durchführung des erfindungsgemäßen Verfahrens geeignet sind, können aus Bodenproben terrestrischen oder marinen Ursprungs durch die üblicherweise zu diesem Zweck angewandten Methoden isoliert werden. Streptomyces griseus Stamm 587 beispielsweise konnte aus einer marinen Sedimentprobe angereichert werden, die mit einem Greifer im Januar 1968 in der Nordsee vor der schottischen Küste aus einer Tiefe von 63 m genommen wurde. Der Stamm wurde seit dieser Zeit auf geeigneten festen Nährböden (z.B. auf HPG-Agar: 3 g Hefeextrakt, 6 g Pepton, 10 g Glucose, 8 g NaCl, 20 g Agar, 1000 ml entmineralisiertes Wasser, pH-Wert auf 7,0 eingestellt) in Kultur gehalten.

Streptomyces griseus-Stamm 587 hat folgende Merkmale:

a) Stamm 587 bildet das für Streptomyceten typische Luftmyzel mit deutlich ausgeprägten Sporenketten. Die Sporen sind oval bis zylindrisch. Abmessungen: 0,4 - 0,8 $\mu$m x 0,8 - 1,8 $\mu$m. Die Sporenoberfläche ist glatt oder geringfügig strukturiert.

b) Die Farbe des jungen Luftmyzels ist weiß. Das ausgereifte Luftmyzel ist gelb bis sandfarben ("griseus").

c) Die Sporophoren sind sympodial verzweigt und in Büscheln angeordnet. Die Sporenketten sind gerade oder gewellt ("rectus flexibilis") und tragen bis zu etwa 60 Sporen.

d) Auf peptonhaltigen Nährböden wird kein dunkles Pigment gebildet. Der Stamm ist daher nicht chromogen.

Diese Merkmale weisen Stamm 587 (DSM 1471) als zur Art Streptomyces griseus Waksman et Henrici gehörig aus (auf der Grundlage von Hütter, R.: Systematik der Streptomyceten, Basel-New York, Karger 1967).

Das erfindungsgemäße Verfahren kann mit Hilfe fester, halbfester oder flüssiger Nährmedien durchgeführt werden. Bevorzugt werden wäßrig-flüssige Nährmedien verwendet.

Die Beimpfung der Nährmedien erfolgt nach allgemein üblichen Methoden, z.B. über Schrägröhrchen oder Kolbenkulturen.

Die Kultur erfolgt unter aeroben Bedingungen und kann gemäß den allgemein üblichen Methoden als Schüttelkultur z.B. in Schüttelkolben, als luftbewegte Kultur oder als Submerskultur durchgeführt werden. Bevorzugt erfolgt die Kultivierung im aeroben Submersverfahren in belüfteten Fermentern, z.B. in üblichen Submersfermentationstanks. Es ist möglich, die Kultur kontinuierlich oder diskontinuierlich durchzuführen. Vorzugsweise wird diskontinuierlich gearbeitet.

Die Kultur kann in allen Nährmedien durchgeführt werden, welche bekannterweise zur Kultivierung von Mikroorganismen der Ordnung Actinomycetales verwendet werden. Das Nährmedium muß eine oder mehrere assimilierbare Kohlenstoffquellen und Stickstoffquellen

sowie Mineralsalze enthalten, wobei diese Produkte in Form von definierten Einzelbestandteilen, aber auch in Form von komplexen Gemischen, wie sie insbesondere biologische Produkte verschiedenen Ursprungs darstellen, vorliegen können. Als Kohlenstoffquellen kommen alle üblichen Kohlenstoffquellen in Frage. Als Beispiel seien Stärke, Melasse, Molkepulver, Dextrin und Zucker, wie Saccharose, Maltose, Glucose, Lactose, Sorbit und Glycerin genannt. Als Stickstoffquellen kommen alle üblichen organischen und anorganischen Stickstoffquellen in Frage. Als Beispiel seien Sojabohnenmehl, Baumwollsamenmehl, Linsenmehl, Erbsenmehl, lösliche und unlösliche pflanzliche Proteine, Maisquellwasser, Hefeextrakt, Peptone und Fleischextrakt sowie Ammoniumsalze und Nitrate, z.B. $NH_4Cl$, $(NH_4)_2SO_4$, $NaNO_3$ und $KNO_3$ aufgeführt. Die Mineralsalze, welche im Nährmedium enthalten sein sollen, liefern z.B. folgende Ionen:

$Mg^{++}$, $Na^+$, $K^+$, $Ca^{++}$, $NH_4^+$ , $Cl^-$, $SO_4^{--}$, $PO_4^{---}$ und $NO_3^-$

sowie Ionen der üblichen Spurenelemente, wie Cu, Fe, Mn, Mo, Zn, Co, Ni. Falls die Kohlenstoff- oder Stickstoffquellen bzw. das verwendete Wasser nicht ausreichend diese Salze bzw. Spurenelemente enthalten, ist es zweckmäßig, das Nährmedium entsprechend zu ergänzen. Die Zusammensetzung der Nährmedien kann in weiten Bereichen variiert werden. Art und Zusammensetzung der Nährmedien werden im allgemeinen davon abhängig sein, welche Bestandteile jeweils besonders günstig zur Verfügung stehen.

Bei der Durchführung des Verfahrens kann es günstig sein, zu Beginn der Kultivierung nur relativ geringe Konzentrationen der löslichen Nährlösungsbestandteile zu verwenden und dann im Laufe der Kultivierung diese Bestandteile in Form steriler, relativ konzentrierter Lösungen dem Kulturansatz fraktioniert zuzufüttern. Der pH-Wert der wachsenden Kulturen sollte zwischen etwa 5,5 und etwa 9,5 gehalten werden. Ein zu starker pH-Abfall in den sauren Bereich kann durch Zusätze einer organischen oder anorganischen Base, vorzugsweise von $CaCO_3$ vermieden werden. Wie in der Fermentationstechnologie üblich, kann auch eine automatische pH-Regulierung durchgeführt werden, bei der sterile organische oder anorganische Säure, z.B. $H_2SO_4$, oder sterile Lauge, z.B. NaOH in Abständen in die Kulturlösung eingespritzt wird.

Es ist zweckmäßig, sicherzustellen, daß die Mikroorganismen ausreichend mit Sauerstoff sowie den Nährstoffen in Kontakt gebracht werden. Dies kann nach den allgemein üblichen Methoden wie Schütteln und Rühren erfolgen.

Die Züchtungstemperatur kann zwischen etwa 10 und etwa 40°C, liegen. Die Dauer der Züchtung kann stark variiert werden, wobei z.B die Zusammensetzung des Nährmediums und die Züchtigungstemperatur eine Rolle spielen. Die jeweiligen optimalen Bedingungen können von jedem Fachmann auf dem mikrobiolgischen Gebiet leicht festgelegt werden.

Es hat sich herausgestellt, daß die Menge der sich in der Kulturbrühe anreichernden Verbindung im allgemeinen ihr Maximum zwischen dem 1. und 12., vorzugsweise zwischen dem 2. bis 5. Tage nach Züchtungsbeginn erreicht.

Wie allgemein bei mikrobiolgischen Verfahren sollten Fremdinfektionen der Kulturmedien vermieden werden. Hierzu werden die üblichen Vorkehrungen getroffen, wie Sterilisation der Nährmedien, der Kulturgefäße sowie der für die Belüftung notwendigen Luft. Zur Sterilisation der Vorrichtungen können z.B. die Dampf- als auch die Trockensterilisation verwendet werden.

Falls bei der Kultivierung in unerwünschter Menge Schaum entsteht, können die üblichen chemischen Schaumdämpfungsmittel, z.B. flüssige Fette und Öle, Öl-Wasser-Emulsionen, Paraffine, höhere Alkohole, wie Octadecanol, Siliconöl, Polyoxyethylen- bzw. Polyoxypropylenverbindungen zugesetzt werden. Schaum kann auch mit Hilfe der üblichen mechanischen Vorrichtungen (welche z.B. Zentrifugalkräfte benutzen) gedämpft oder beseitigt werden.

Die Verbindung kann aus dem Kulturmedium nach allgemein üblichen physikalisch-chemischen Methoden isoliert werden. Die Isolierung kann z.B. gemäß den üblichen Extraktionsverfahren, Fällungsverfahren und/oder Chromatographieverfahren erfolgen. Das isolierte Herbizid kann mit Hilfe der genannten Methoden auch feingereinigt werden. Für viele Fälle ist jedoch eine Feinreinigung nicht erforderlich, da die gegebenenfalls vorhandenen Verunreinigungen die Wirksamkeit der Verbindung als Herbizid nicht nachteilig beeinflussen. Bei allen Isolierungs- und Reinigungsoperationen ist darauf zu achten, daß pH-Werte oberhalb pH 2 eingehalten werden. Zur Erhöhung des pH-Wertes können anorganische und organische Basen verwendet werden, z.B. Ammoniak, Alkali- und Erdalkalihydroxide, Alkali- und Erdalkalicarbonate und Hydrogencarbonate, z.B. KOH, NaOH, $Na_2CO_3$, $NaHCO_3$, $CaCO_3$, Trialkylamine wie Triethylamin, Morpholin und Pyridin. Um bei den obengenannten Isolierungs- und Reinigungsmethoden die Fraktionen herauszufinden, in welchen die erfindungsgemäße Verbindung in höchster Konzentration bzw. Reinheit vorliegt, können die üblichen physikalisch-chemischen Methoden, z.B. Messen der $R_f$-Werte, oder aber die Untersuchung der herbiziden Aktivität und vorzugsweise die Bestimmung der antibiotischen Aktivität herangezogen werden.

Die Isolierung der Verbindung kann z.B. im Falle, daß ein flüssiges

wäßriges Nährmedium verwendet wird, wie folgt vorgenommen werden:

Nach seiner Anreicherung im Kulturüberstand werden Kulturfiltrat und Mycel mit üblichen Methoden separiert (z.B. Zentrifugation).

Aus dem Kulturfiltrat kann die Verbindung mit Hilfe von üblichen Extraktionsverfahren, Fällungsverfahren und/oder Chromatographieverfahren isoliert und gegebenenfalls gereinigt werden. Die Chromatographie kann in Form der Säulenchromatographie durchgeführt werden. Als Adsorptionsmittel können die üblichen anorganischen oder organischen Adsorptionsmittel eingesetzt werden, wie z.B. Aluminiumoxid, Kieselgel, Magnesiumsilikat, Aktivkohle, Cellulose, Cellulosederivate, Kunstharze wie Polyamide, Derivate von Polyamiden, z.B. acetyliertes Polyamid oder Dextrangele. Als Laufmittel können die verschiedensten Lösungsmittel oder Lösungsmittelgemische verwendet werden, in welchen die erfindungsgemäße Verbindung löslich ist. Bevorzugt wird Methanol und Ethanol eingesetzt.

Bevorzugt werden zur Isolierung der Verbindung Chromatographie-Verfahren verwendet, z.B. unspezifische Adsorption an hydrophobe Sorbentien. Dies sind die von der Reinigung schwer wasserlöslicher neutraler Naturstoffe her bekannten Methoden.

Für die kommerzielle Herstellung der als Herbizid oder Pflanzenwachstumsregulator verwendeten Verbindung bevorzugt man die Gewinnung durch Absorption und anschließende Desorption an einem hydrophoben Trägerharz (z.B. Lewapol; ein hydrophobes Trägerharz der BAYER AG). Die Desorption kann z.B. mit kurzkettigen aliphatischen Alkoholen durchgeführt werden, bevorzugt Methanol oder Ethanol.

Eine derart vorgereinigte Fraktion kann wiederum mit den üblichen Methoden gereinigt werden. Bevorzugt kann hier die Geldiffusionschromatographie eingesetzt werden. Die Chromatographie beispielsweise an Sephadex LH-20 verläuft erfolgreich. Vorzugsweise wird hier in alkoholischer Lösung gearbeitet. Ein derartig gewonnenes Produkt ist im allgemeinen reiner als 50 %.

Die Verbindung kann aus einer solchen Fraktion mit den üblichen biochemischen Methoden gewonnen werden. Von den bereits genannten Adsorptionsmitteln kann neben anderen vor allem Kieselgel erfolgreich eingesetzt werden. Als Fließmittel kommt z.B. Chloroform-Methanol (so etwa 4/1 Volumenteile) mit Erfolg zur Anwendung.

Aus seinen Lösungen kann die Verbindung nach den üblichen Methoden, z.B. Verdampfen des Lösungsmittels, Gefriertrocknung usw. erhalten werden.

Der erfindungsgemäß hergestellte Wirkstoff beeinflußt das Pflanzenwachstum und kann deshalb als Defoliant,

Desiccant, Krautabtötungsmittel, Keimhemmungsmittel und insbesondere als Unkrautvernichtungsmittel, jedoch auch bevorzugt als Pflanzenwachstumsregulator verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Der erfindungsgemäß hergestellte Wirkstoff kann z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monocharia, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung des Wirkstoffes ist jedoch keineswegs auf diese Gattungen beschränkt, sondern erstreckt sich in gleicher Weise auch auf andere Pflanzen.

Die Verbindung eignet sich in Abhängigkeit von der Konzentration zur Totalunkrautbekämpfung z.B. auf Industrie- und Gleisanlagen und auf Wegen und Plätzen mit und ohne Baumbewuchs. Ebenso kann die Verbindung zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Der Wirkstoff kann als solcher oder in Formulierungen auch in Mischung mit bekannten Herbiziden zur Unkrautbekämpfung Verwendung finden, wobei Fertigformulierung oder Tankmischung möglich ist.

Der Wirkstoff kann in die üblichen Formulierungen übergeführt werden wie Lösungen, Emulsionen, Spritzpulver, Suspensionen, Pulver, Stäubemittel, Pasten, lösliche Pulver, Granulate, Suspensions-Emulsionskonzentrate, Wirkstoff-imprägnierte Natur- und synthetische Stoffe, Feinstverkapselungen in polymeren Stoffen.

Diese Formulierungen werden in bekannter Weise hergestellt, z.B. durch Vermichen des Wirkstoffs mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage:

Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

Natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyethylen-Fettsäure-Ester, Polyoxyethylen-Fettalkohol-Ether, z.B. Alkylaryl-polyglykol-ether, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salz von Eisen, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß erhältliche Verbindung eignet sich besonders gut auch zur Fruchtablösung (Ernteerleichterung), insbesondere bei Citrusfrüchten und Oliven.

Beispiel 1

Beimpft man einen 1 l Erlenmeyerkolben, der 150 ml einer Nährlösung der Zusammensetzung 0,5 % $(NH_4)_2SO_4$, 0,3 % $KNO_3$, 0,4 % Sojamehl, 0,4 % Maisquellwasser, 6,0 % Stärke, 0,5 % $CaCO_3$ (pH-Einstellung vor Sterilisation auf 7,0 mit KOH, Sterilisation 20 Minuten 121°C) enthält mit 3 ml einer Vorkultur des Stammes 587 (gewonnen in derselben Nährlösung, beimpft mit einer Schrägröhrchenkultur) und bebrütet diese Kultur bei 28°C auf einer Rundschüttelmaschine bei 280 Upm, so erhält man nach einer Kulturdauer von 3 Tagen eine Kulturbrühe, deren Gehalt an Wirkstoff gemäß Beispiel 9 zu 32 mg/l bestimmt wird.

Beispiel 2

Beimpft man einen 1 l Erlenmeyerkolben, der 150 ml einer Nährlösung aus 2 % Stärke, 1 % Glucose, 0,5 % N-Z Amine, 1,0 % Hefeextrakt enthält, deren pH-Wert mit $Na_2CO_3$ auf 7,2 eingestellt und die dann mit 0,4 % $CaCO_3$ versetzt und 30 Minuten bei 121°C sterilisiert worden war, mit 3 ml einer Vorkultur des Stammes 587, gewonnen in derselben Nährlösung, und bebrütet auf einer Rundschüttelmaschine bei 28°C und 280 Upm, so erhält man nach 2 Tagen eine Kulturbrühe, deren Gehalt an Wirkstoff gemäß Beispiel 9 zu 4 mg/l bestimmt wird. (N-Z-Amine = Eiweißhydrolysate).

Beispiel 3

Beimpft man einen 1 l Erlenmeyerkolben, der 150 ml einer

Nährlösung aus 3 % Sojamehl entfettet, 3 % Glyzerin, 0,2 % $CaCO_3$, 0,1 % Polyol-Entschäumer enthält und 30 Minuten bei 121°C sterilisiert worden war, mit 3 ml eine Vorkultur des Stammes 587, gewonnen in derselben Nährlösung, und bebrütet bei 28°C auf einer Rundschüttelmaschine bei 280 Upm, so erhält man nach 3 Tagen eine Kulturbrühe, deren Gehalt an Wirkstoff gemäß Beispiel 9 zu 8 mg/l bestimmt wird.

Beispiel 4

Beimpft man einen 1 l Erlenmeyerkolben, der 150 ml einer Nährlösung aus 3 % Stärke, 2 % Hefeautolysat, 0,1 % $K_2HPO_4$, 0,1 % $MgSO_4 \cdot 7H_2O$, 0,1 % NaCl, 0,2 % $(NH_4)_2SO_4$ und 0,2 % $CaCO_3$ enthält, deren pH-Wert auf 7,0 eingestellt und die 30 Minuten bei 121°C autoklaviert worden war, mit 3 ml einer Vorkultur, gewonnen in derselben Nährlösung, und bebrütet auf einer Rundschüttelmaschine, bei 28°C und 280 Upm, so erhält man nach 3 Tagen eine Kulturbrühe, deren Gehalt an Wirkstoff gemäß Beispiel 9 zu 4 mg/l bestimmt wird.

Beispiel 5

Beimpft man einen Fermenter, der 200 l einer Nährlösung nach Beispiel 1 enthält, mit 10 l einer in einem Vorfermenter gewonnenen Vorkultur des Stammes 587 und bebrütet unter Rührung (200 Upm) und Belüftung (0,5 vvm) 5 Tage bei 28°C, so erhält man eine Kulturbrühe, deren Gehalt an Wirkstoff gemäß Beispiel 9 zu 128 mg/l bestimmt wird.

Beispiel 6

Bei einer Fermentation gemäß Beispiel 5 werden nach dem Abzentrifugieren 170 l Kulturfiltrat gewonnen. Der Kulturüberstand wird mit 50 l/h auf eine Säule (30x50 cm), die ein hydrophobes Trägerharz auf Sulfonsäurebasis (z.B. Lewapol) enthält, aufgetragen und die Säule mit 100 l $H_2O$ nachgewaschen. Durchlauf und Waschwasser sind nicht herbizid wirksam und werden verworfen. Anschließend wird zunächst mit 100 l 50 %igem Methanol desorbiert. Das inaktive Desorbat wird verworfen. Anschließende Desorption mit 100 l 100 %igem Methanol führt zu einem herbizid wirksamen Eluat. Das Eluat wird zur Trockene eingeengt, der so erhaltene Rückstand in 2 l Wasser aufgenommen und die Suspension lyophilisiert (Ausbeute 40,2 g 3,5 %iger Wirkstoff).

Beispiel 7

Man löst 4,0 g des 3,5 %igen Wirkstoffs gemäß Beispiel 6 in 50 ml Methanol, zentrifugiert ab und chromatographiert den Überstand an Sephadex LH-20 in Methanol (Säule 5 cm x 100 cm, Fließrate 100 ml/h, 12'/Glas). Die Fraktionen 75-78 zeigen Hemmwirkung gegen die Hefe HT-21 und sind auch herbizid wirksam (Ausbeute 119 mg). Der Wirkstoffgehalt läßt sich gemäß Beispiel 9 zu ca. 50 % bestimmen.

Beispiel 8

Man löst 100 mg angereichertes Präparat gemäß Beispiel 7 in 5 ml Chloroform/Methanol = 5/1 (v/v) und trägt linien-

förmig auf eine präparative 2 mm dicke Kieselgelplatte (KG 60, Merck) auf. Es wird mit dem angegebenen Fließmittel eluiert. Die Zone mit einem $R_f$-Wert von 0,55 bis 0,75 wird mit Methanol eluiert und enthält 27,7 mg des reinen Wirkstoffs.

Beispiel 9

Aus einer Lösung von 1 mg/ml des reinen Wirkstoffs gemäß Beispiel 8 wird im Lochtest die Hemmpotenz gegen die Hefe HT-21 bestimmt. Dabei wird nach 12 Stunden Inkubation bei 28°C der Durchmesser des Hemmhofes in Abhängigkeit von der Konzentration gemessen.

| 1 mg/ml Wirkstoff | unverdünnt | 1:1 | 1:4 | 1:8 | 1:16 |
|---|---|---|---|---|---|
| Ø Hemmhof mm | 59 | 49 | 45 | 43 | 42 |

| 1:32 | 1:64 | 1:128 | 1:256 | 1:512 | 1:1024 | 1:2048 | 1:4096 |
|---|---|---|---|---|---|---|---|
| 40 | 35 | 30 | 23 | 19 | 14 | 12 | - |

Durch Bestimmung der minimalen Hemmkonzentration der Präparate der Beispiele 1 bis 8 konnte daher der Wirkstoffgehalt bestimmt werden.

Beispiel 10

pre-emergence-Test

In Schalen, die mit Vermiculite gefüllt sind, werden

Samen von Kresse (Lepidium sativium) ausgelegt. Die Schalen werden dann mit einer Hoagland-Nährlösung gegossen, der die erfindungsgemäßen Wirkstoffe zugesetzt sind.

Nach 2 Wochen wird der Schädigungsgrad der Pflanzen bonitiert in % Schädigung im Vergleich zur Entwicklung der unbehandelten Kontrolle.

Die Präparate gemäß den obigen Beispielen zeigen eine hohe Wirksamkeit.

Beispiel 11

post-emergence-Test

In Schalen, die mit Vermiculite gefüllt sind, werden Samen von Kresse (Lepidium sativum) ausgelegt. Die Schalen werden dann mit einer Hoagland-Nährlösung gegossen, bis die Pflanzen eine Größe von 5 bis 10 cm erreicht haben.

Dann werden die Pflanzen mit einer Wirkstoffzubereitung gespritzt.

Nach 2 Wochen wird der Schädigungsgrad der Pflanzen bonitiert in % Schädigung im Vergleich zur Entwicklung der unbehandelten Kontrolle.

Die Präparate gemäß den obigen Beispielen zeigen eine hohe Wirksamkeit.

Beispiel 12

Pre-emergence-Test

Lösungsmittel: 5 Gewichtsteile Aceton
Emulgator: 1 Gewichtsteil Alkylarylpolyglykolether
Wst: Wirkstoff

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach 3 Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Bei diesem Test weist der erfindungsgemäße Wirkstoff gegenüber dem Stand der Technik deutliche Vorteile auf.

Beispiel 13

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, daß die in der Tabelle angegebenen Wirkstoffmengen pro Flächeneinheit ausgebracht werden. Die Konzentration der Spritzbrühe wird so gewählt, daß in 2000 l Wasser/ha die in der Tabelle angegebenen Wirkstoffmenge ausgebracht werden. Nach 3 Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Bei diesem Test weist der erfindungsgemäße Wirkstoff gegenüber dem Stand der Technik deutliche Vorteile auf.

Patentansprüche

1. Verfahren zur Herstellung von 1R;3S;S;αR; 3-[2-(2,5- Dimethyl-2-oxocyclohexyl)-2-hydroxyethyl]-2,6-piperidindion, dadurch gekennzeichnet, daß man Mikroorganismen der Ordnung Actinomycetales in einem Nährmedium, welches assimilierbare Kohlenstoff- und Stickstoffquellen sowie Mineralsalze enthält, unter aeroben Bedingungen kultiviert und die Verbindung isoliert.

2. Verfahren nach Anspruch 1, dadurch gekennzeichnet, daß man Mikroorganismen der Familie Streptomycetaceae kultiviert.

3. Verfahren nach Anspruch 2, dadurch gekennzeichnet, daß man einen Mikroorganismus der Gattung Streptomyces kultiviert.

4. Verfahren nach Anspruch 3, dadurch gekennzeichnet, daß man Streptomyces griseus Stamm 587 (DSM 1471) kultiviert.

5. Herbizides Mittel, enthaltend 1R;3S;5S;αR; 3-[2-(2,5- Dimethyl-2-oxocyclohexyl)-2-hydroxy-ethyl]-2,6-piperidindion.

6. Verwendung von 1R;3S;5S;αR; 3-[2-(3,5- Dimethtyl-2-oxocyclohexyl)-2-hydroxyethyl]-2,6-piperidindion zur Bekämpfung von unerwünschtem Pflanzenwachstum, zur Regulierung von Pflanzenwachstum und zur Fruchtablösung.

7. Verwendung von 1R;3S,5S;αR; 3-[2-(3,5-Dimethyl-2-oxocyclohexyl)-2-hydroxyethyl]-2,6-piperidindion zur Fruchtablösung.

8. Verfahren zur Herstellung eines herbiziden Mittels, dadurch gekennzeichnet, daß man 1R;3S;5S; αR;3-[2-(3,5- Dimethyl-2-oxocyclohexyl)-2-hydroxyethyl]-2,6-piperidindion mit Streckmitteln und/oder oberflächenaktiven Mitteln vermischt.

9. Mikroorganismen der Ordnung Actinomycetales, die bei der Kultivierung in einem Kohlenstoff- und Stickstoffquellen sowie Mineralsalze enthaltendem Nährmedium 1R;3S;5S; αR; 3-[2-(3,5- Dimethyl-2-oxocyclohexyl)-2-hydroxyethyl]-2,6-piperidindion produzieren.

10. Mikroorganismen gemäß Anspruch 9 der Familie Streptomycetaceae.

11. Mikroorganismen gemäß Anspruch 9 der Gattung Streptomyces.

12. Streptomyces griseus Stamm 587 (DSM 1471).

13. Kultur, die einen Mikroorganismus gemäß den Ansprüchen 9 bis 12 enthält.

14. Verwendung von Mikroorganismen bei der Herstellung von 1R,3S;5S; αR; 3-[2-(3,5-Trimethyl-2-oxocyclohexyl)-2-hydroxyethyl]-2,6-piperidindion.

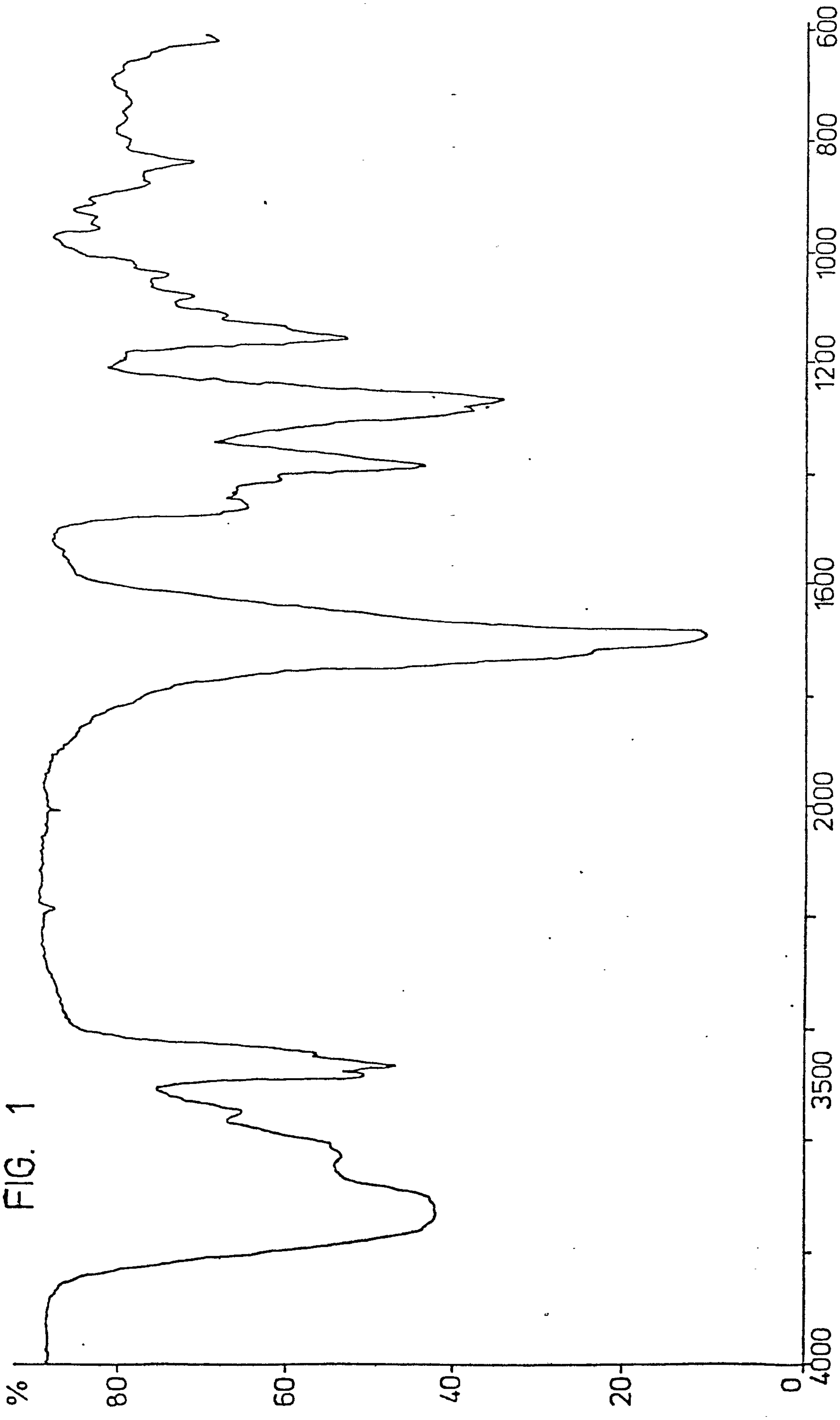


FIG. 1

0022910

FIG 2

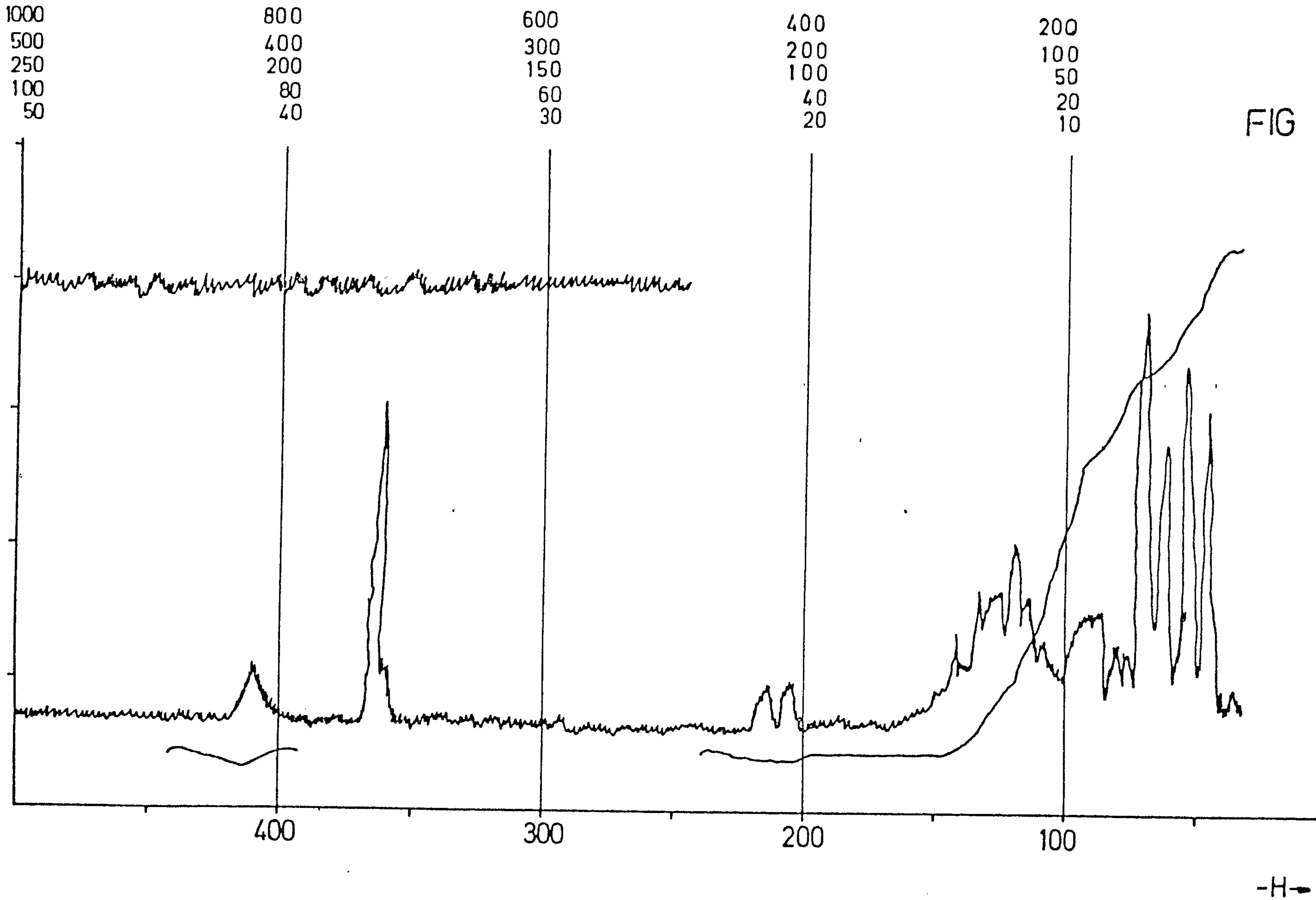

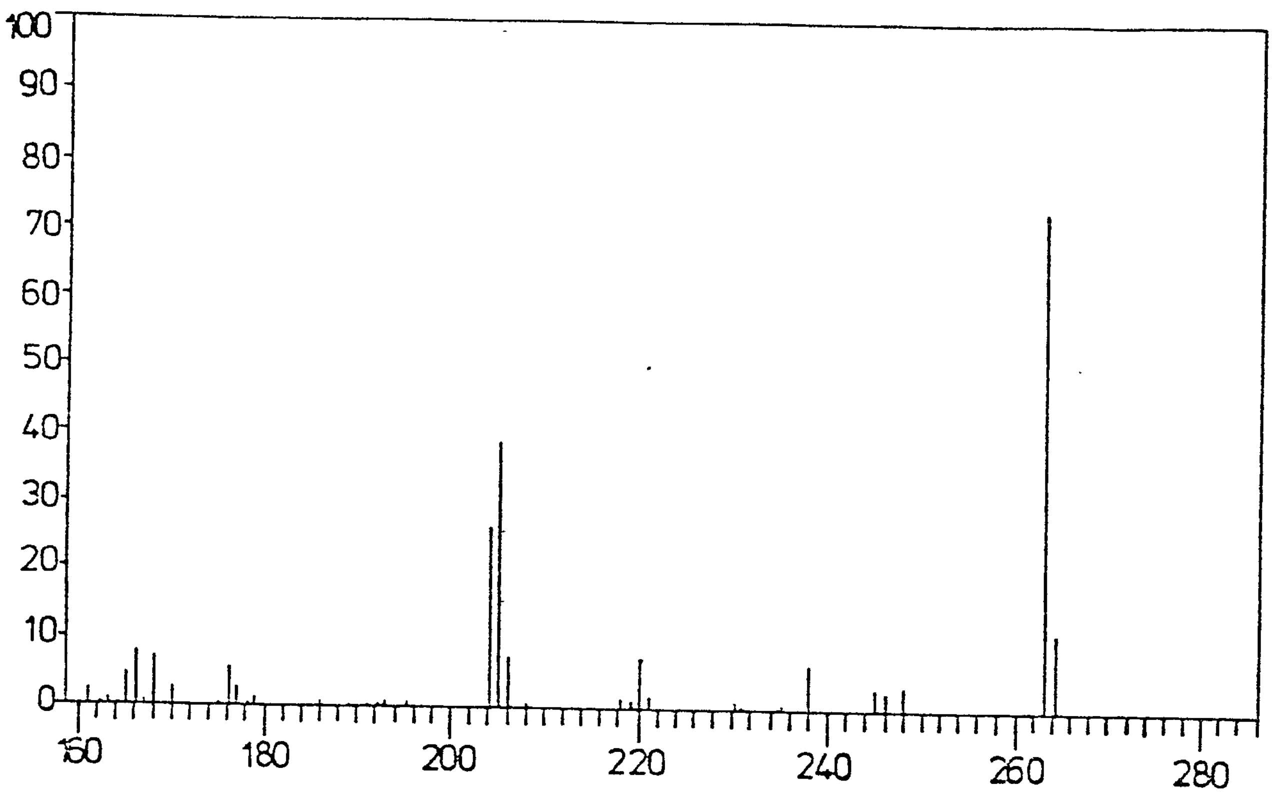
100
90
80
70
60
50
40
30
20
10
0
160
180
200
220
240
260
280

FIG.3

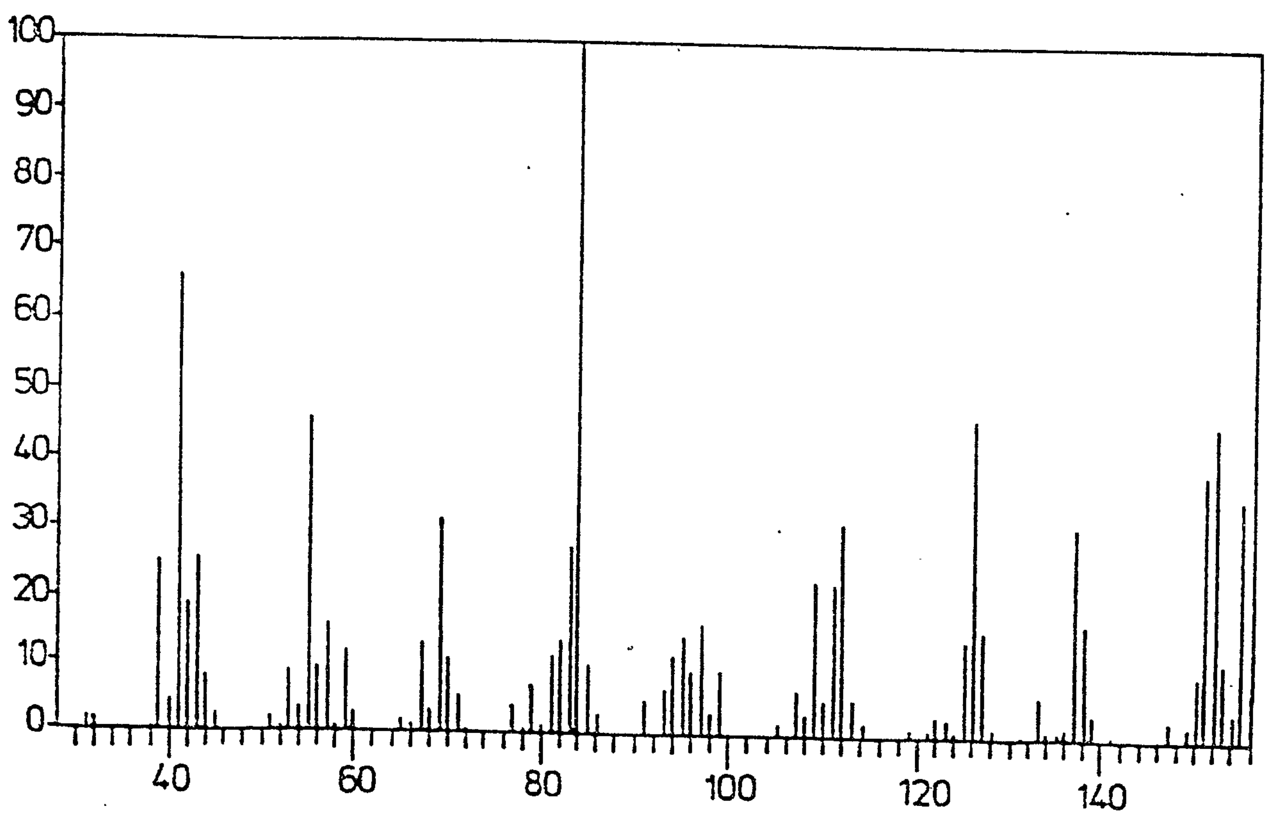
100
90
80
70
60
50
40
30
20
10
0
40
60
80
100
120
140

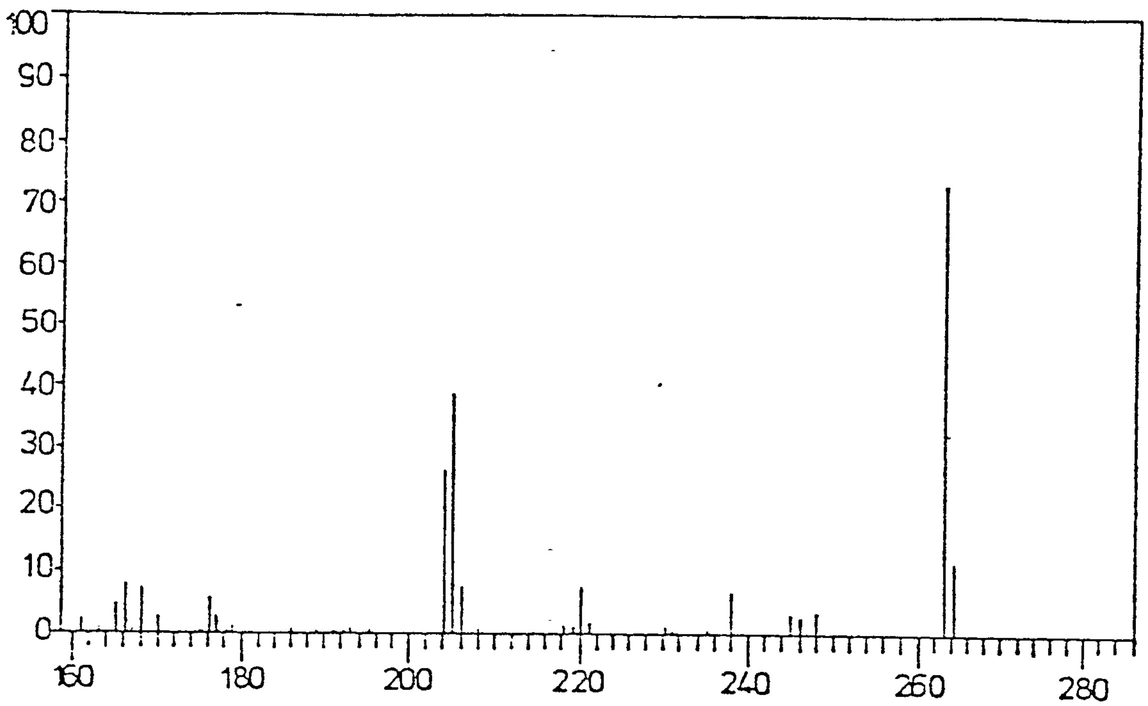
100
90
80
70
60
50
40
30
20
10
0
160
180
200
220
240
260
280

FIG. 4

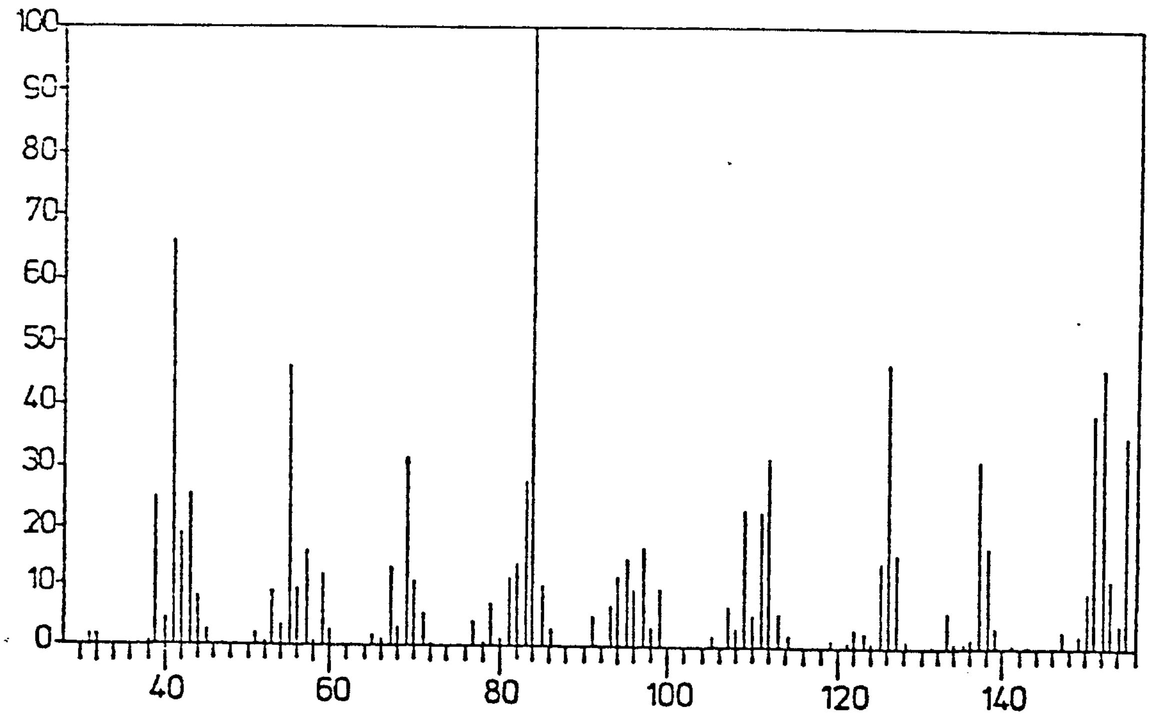
100
90
80
70
60
50
40
30
20
10
0
40
60
80
100
120
140

5/5

0022910

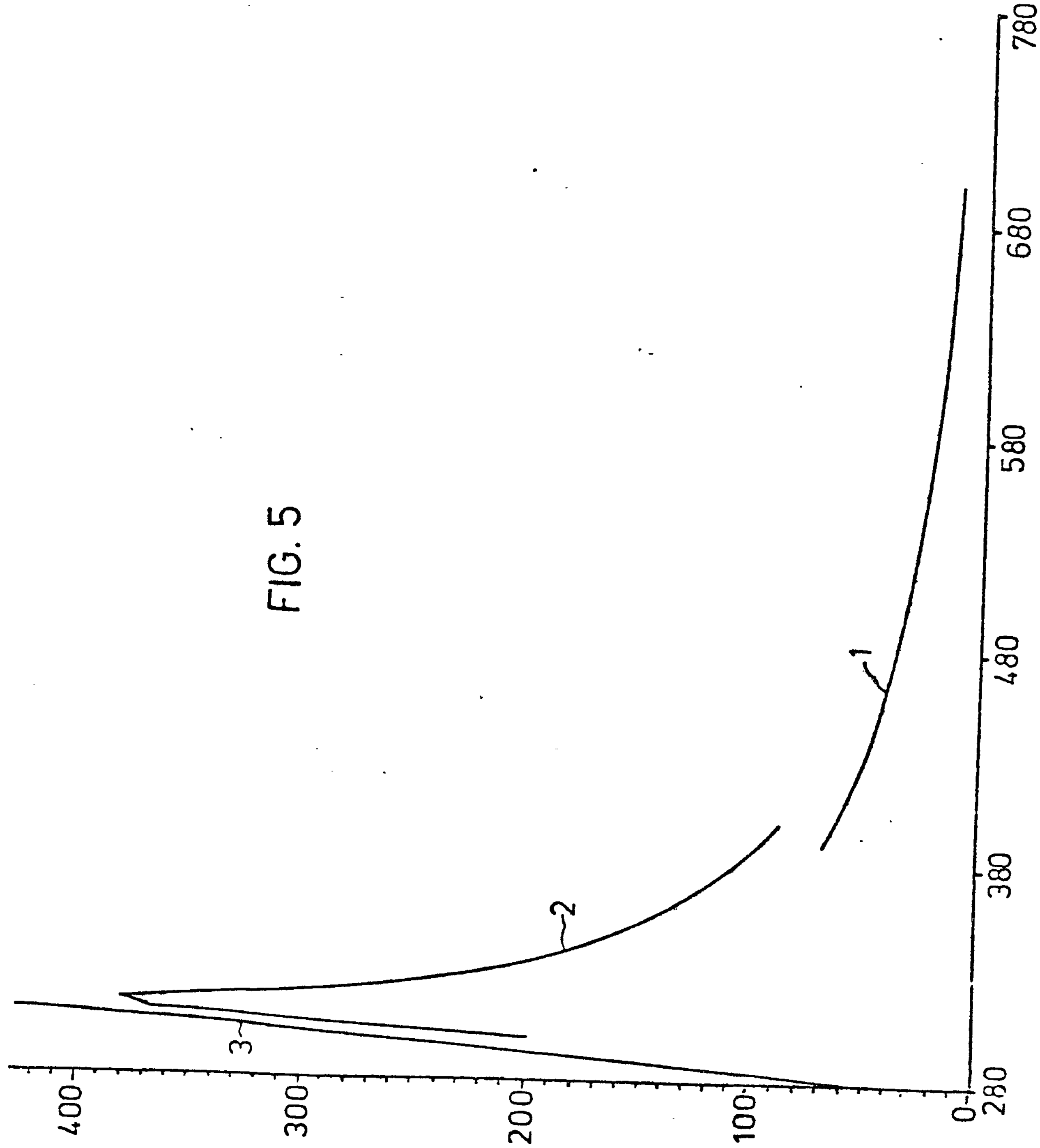


FIG. 5

0022910

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 80 10 2819

## EINSCHLÄGIGE DOKUMENTE

| Kategorie | Kennzeichnung des Dokuments mit Angabe, soweit erforderlich, der maßgeblichen Teile | betrifft Anspruch |
| --- | --- | --- |
| X | FR - A - 1 274 358 (UPJOHN)<br>* Insgesamt *<br>-- | 1-5,9-11,13,14 |
| X | H. UMEZAWA: "Index of Antibiotics from Actinomycetes", Band 1, 1967, Seite 237, University of Tokyo Press<br>Tokyo, JP.<br>"Cycloheximide"<br>* Seite 237 *<br>-- | 1-5,9-11,13,14 |
| X | CHEMICAL ABSTRACTS, Band 90, Nr. 25, 18. Juni 1979, Seite 473, Nr. 202240x<br>Columbus, Ohio, U.S.A.<br>& JP - A - 78 139 790 (GODO SHUSEI CO., LTD.; NISSAN CHEMICAL INDUSTRIES, LTD.) 06-12-1978<br>* Zusammenfassung *<br>-- | 1-5,9-11,13,14 |
| | THE JOURNAL OF ORGANIC CHEMISTRY, Band 25, Nr. 2, 21. März 1960, Seiten 344-346<br>./. | 1 |

KLASSIFIKATION DER ANMELDUNG (Int. Cl.³)

C 12 P 17/12
C 07 D 211/88
A 01 N 43/40//
(C 12 P 17/12
C 12 R 1/545)

RECHERCHIERTE SACHGEBIETE (Int. Cl.³)

C 12 P 17/12
C 07 D 211/88
A 01 N 43/40//
(C 12 P 17/12
C 12 R 1/545)

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-8, 13,14

Unvollständig recherchierte Patentansprüche:

Nicht recherchierte Patentansprüche: 9-12 (Art. 53(b) des Europäischen Patentübereinkommens.)

Grund für die Beschränkung der Recherche:

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
| --- | --- | --- |
| Den Haag | 05-09-1980 | RAJIC |

EPA Form 1505.1 06.78

Europäisches Patentamt

Nummer der Anmeldung
EP 80 10 2819

# EUROPÄISCHER TEILRECHERCHENBERICHT



| EINSCHLÄGIGE DOKUMENTE | | | KLASSIFIKATION DER ANMELDUNG (Int Cl.³) |
|---|---|---|---|
| Kategorie | Kennzeichnung des Dokuments mit Angabe, soweit erforderlich, der maßgeblichen Teile | betrifft Anspruch | |
| | American Chemical Society<br>A.J. LEMIN et al.: "Isocyclohexi-mide"<br>* Insgesamt *<br>-- | | |
| | GB - A - 799 731 (UPJOHN)<br>* Insgesamt *<br>-- | 1-6,8 | |
| | JOURNAL OF THE AMERICAN CHEMICAL SOCIETY, Band 86, Nr. 1, 5. Januar 1964<br>Seiten 118-119<br>Columbus, Ohio, U.S.A.<br>F. JOHNSON et al.: "Glutarimide Antibiotics. IV. The Total Synthesis of dl- and l-Cycloheximide"<br>* Insgesamt *<br>-- | 1 | RECHERCHIERTE SACHGEBIETE (Int. Cl.³) |
| | FR - A - 1 352 101 (TANABE)<br>* Insgesamt *<br>---- | 1-6,9-11,13,14 | |